Europäisches Patentamt

European Patent Office

Office européen des brevets

(11) Publication number: **0 426 084 B1**

# (12) EUROPEAN PATENT SPECIFICATION

(45) Date of publication of patent specification :
27.01.93 Bulletin 93/04

(51) Int. Cl.⁵: **C07C 291/04**

(21) Application number : **90120733.2**

(22) Date of filing : **29.10.90**

(54) **Amine oxide process.**

(30) Priority : **30.10.89 US 429032**
**20.08.90 US 569648**

(43) Date of publication of application :
**08.05.91 Bulletin 91/19**

(45) Publication of the grant of the patent :
**27.01.93 Bulletin 93/04**

(84) Designated Contracting States :
**DE FR GB IT**

(56) References cited :
**GB-A- 2 032 422**

(73) Proprietor : **ETHYL CORPORATION**
**Ethyl Tower 451 Florida Boulevard**
**Baton Rouge Louisiana 70801 (US)**

(72) Inventor : **Borland, James Ellwood**
**12253 East Millburn**
**Baton Rouge, Louisiana 70815 (US)**
Inventor : **Impastato, Fred John**
**1164 Ashbourne Drive**
**Baton Rouge, Louisiana 70815 (US)**
Inventor : **Smith, Kim Renae**
**1950 Stafford Drive**
**Baton Rouge, Louisiana 70810 (US)**

(74) Representative : **Sandmair, Kurt, Dr. et al**
**Patentanwälte Schwabe, Sandmair, Marx**
**Stuntzstrasse 16**
**W-8000 München 80 (DE)**

EP 0 426 084 B1

Jouve, 18, rue Saint-Denis, 75001 PARIS

## Description

This invention relates to amine oxides and more particularly to a method of minimizing the discoloration of amine oxides prepared by high solids processes in the presence of a certain amount of a non-reactive gas and carbon dioxide.

Amine oxides are materials which have been found to have a variety of applications and have been used, e.g., in the treatment of fabrics and in the preparation of hair conditioners and shampoos, toothpaste, laundry detergent powders, fabric softeners, toilet soap bars, and cosmetics, as well as in other applications.

When synthesized in the conventional manner so as to be provided as dilute solutions, the amine oxides have acceptable color, even when they are prepared in the presence of carbon dioxide, as in U. S. Patent 4,247,480 (Murata et al.) and European Patent Application 0320694 (Bauer et al.).

This is not the case, however, when the amine oxides are prepared by the newer high solids processes. When amine oxides are prepared by the reaction of tert-amines with hydrogen peroxide in a liquid medium which constitutes not more than 50% of the weight of the reaction mixture, they have acceptable color when the reaction is conducted in the absence of carbon dioxide; but they are intensely colored when carbon dioxide is used to speed the reaction.

It has now been found that, when an amine oxide is prepared by reacting a tert-amine with hydrogen peroxide at a temperature of 20-100°C in the presence of carbon dioxide and in a liquid medium that constitutes not more than 50% of the weight of the reaction mixture, discoloration of the product can be minimized by conducting the reaction in the presence of an amount of non-reactive gas wherein the amount is such as to constitute 60-75 % of the combined volumes of non-reactive gas and carbon dioxide.

The high solids process which is modified in accordance with the present invention is one in which the amount of liquid medium employed is minimized to provide a product which is a solid or a concentrated solution.

In all of the high solids processes, the reaction mixture contains the water contributed by the aqueous hydrogen peroxide, as well as the water formed by the reaction; and this water may be the only liquid medium used when it is sufficient to keep the reaction mixture fluid and stirrable. However, an organic solvent and/or additional water may be used to maintain stirrability when appropriate as long as the total amount of liquid medium is not allowed to exceed 50% of the weight of the reaction mixture.

When an organic solvent is utilized, it is preferably an organic solvent in which the tert-amine and amine oxide are soluble at the reaction temperatures but in which the amine oxide is insoluble at a lower temperature, usually a substantially inert ester, hydrocarbon, halohydrocarbon, or highly polar aprotic solvent. This solvent, when employed, is preferably used only in the amount required to maintain a stirrable reaction mixture; and it is generally added to the reaction mixture only as needed, although the total amount to be used can be included in the initial reaction mixture if desired.

Exemplary of the organic solvents that are used in these processes are the ethyl, butyl, and sec-butyl acetates, methyl propionate, methyl benzoate, toluene, heptane, N,N-dimethylformamide, and N,N-dimethylacetamide. Ethyl acetate is apt to be especially preferred.

The amine which is oxidized in the process may be any of the amines conventionally used in such processes. As is known, these amines include a variety of tert-amines having aliphatic, cycloaliphatic, and/or aromatic groups attached to the amine nitrogen. However, they are generally trialkylamines corresponding to the formula RR'R"N wherein R, R', and R" are primary alkyl groups containing 1-30 carbons, preferably such trialkylamines in which R is methyl or ethyl, R' is an alkyl group containing 6-20 carbons, and R" is independently selected from methyl, ethyl, and alkyl groups containing 6-20 carbons. Those which are used in the processes in which the liquid medium is water are the trialkylamines in which R is methyl or ethyl and R' and R" are independently selected from alkyl groups containing 6-20 carbons.

Exemplary of the tert-amines that may be used are trimethylamine, triethylamine, N-isobutyldimethylamine, trihexylamine, N,N-dimethyl-2-ethylhexylamine, N-eicosyldimethylamine, N-isobutyl-N-triacontylmethylamine, N-benzyldimethylamine, N-ethyldibenzylamine, N,N-diisobutyl-4-t-butylbenzylamine, tri-2-hydroxyethylamine, and, more preferably, the N-alkyldimethyl- and N,N-dialkylmethylamines in which the alkyl groups are hexyl, octyl, decyl, dodecyl, tetradecyl, hexadecyl, octadecyl, and/or eicosyl, as well as mixtures of such amines.

The hydrogen peroxide which is reacted with the tert- amine is generally an aqueous hydrogen peroxide having an initial concentration of 50-99%, preferably 50-70%; and the amount employed is usually at least the stoichiometric amount but not more than a 20% molar excess. Somewhat less concentrated solutions, i. e., aqueous solutions having a concentration as low as 40%, are sometimes used in the processes in which the liquid medium is water and can also be used in the processes in which the liquid medium comprises an organic solvent. However, it is more common to use an aqueous hydrogen peroxide having a concentration of about 70% in the latter type of process since the use of such a solution is conducive to the easy formation of a final reaction mixture in which the water/amine oxide mol ratio is in the range of 1.9-2.1/1 and therefore can obviate the need for adjusting this ratio at the end of the reaction when the process is used to prepare solid amine oxide dihydrates.

Regardless of which type of liquid medium is used, the amine oxide synthesis is conducted by adding aqueous hydrogen peroxide to the stirred tert-amine, preferably at a controlled rate, in the presence of carbon dioxide and the inert gas and preferably also in the presence of a chelating agent, such as diethylenetriaminepentaacetic acid or ethylenediaminetetraacetic acid, at a temperature in the range of 20-100°C, preferably 60-80°C, a temperature which is maintained for 1-24 hours before the reaction mixture is cooled.

The carbon dioxide is employed in an amount sufficient to speed the reaction, generally at least 0.005%, preferably at least 0.01%, based on the weight of the tert-amine; and this amount can range up to the solubility limit of carbon dioxide in the tert-amine as long as it does not constitute a volume that exceeds the volume of non-reactive gas used.

The non-reactive gas that is used in conjunction with the carbon dioxide may be any of the gases that are conventionally used to provide non-reactive atmospheres for reactions which are poisoned by oxygen or air. However, since the process of the invention is an oxidation process utilizing an oxidizing agent which is even more potent that oxygen or air, and the function of the non-reactive gas appears to be solely to dilute the carbon dioxide rather than to exclude an oxidizing atmosphere that naturally would not have any deleterious effect on the oxidation reaction, the non-reactive gas may be any gas that is inert to the other components of the reaction mixture. In fact, air itself is one of the non-reactive gases that can be used effectively. Other useful non-reactive gases include nitrogen, argon, helium, methane, ethane, propane, ethylene, and the like, with the preferred non-reactive gas being nitrogen because of its availability and low cost.

The carbon dioxide and non-reactive gas may be blended with one another before being fed into the reaction vessel, or they may be introduced separately when the introduction is accomplished in a manner that avoids contacting the reaction mixture with carbon dioxide that is undiluted with the non-reactive gas, e.g., by injecting the separate gases into the vapor space above the reaction mixture. When the carbon dioxide and non-reactive gas are preblended, the mixture may be injected into the liquid phase and/or the vapor phase; and it is effective either to pass the mixture continuously over or through the reaction mixture or to provide the reactor with a carbon dioxide non-reactive gas atmosphere initially and then seal the reactor.

As already mentioned, the amount of non-reactive gas used is such as to constitute 60-99%, most preferably 60-75%, of the combined volumes of non-reactive gas and carbon dioxide.

The amine oxides which are formed as concentrated aqueous solutions by the process of the invention are normally useful per se and have the advantage of containing less water than amine oxides formed by conventional low solids processes and having better color than amine oxides formed by high solids processes in the presence of carbon dioxide but in the absence of the non-reactive gas.

The amine oxides which are formed as concentrated solutions in a mixture of organic solvent and water are normally recovered before being stored, shipped, or used in their final application. Since the high solids process used to synthesize them is of particular interest for the preparation of solid amine oxide dihydrates, it is customary to adjust the water content of the reaction mixture at the end of the reaction, when necessary, to provide a water/amine oxide mol ratio in the range of 1.9-2.1/1 before separating the amine oxide from the organic solvent. However, this adjustment, which could involve either distilling some water from the reaction mixture or adding water to it, is not required when the reaction mixture already contains the right amount of water to provide the desired water/amine oxide mol ratio whether that ratio be the ratio appropriate for the formation of a dihydrate, a lower ratio appropriate for the formation of a monohydrate, or a higher ratio.

In the high solids processes using an organic solvent, the organic solvent may be removed by distillation. However, as indicated by the type of solvent preferred for use in the process, it is ordinarily preferred to recover the product by cooling the reaction mixture to a temperature at which the amine oxide product is no longer soluble in the solvent and thus to precipitate it. To improve the purity of the product, additional organic solvent may be added to the reaction mixture before it is cooled and/or the product may be recrystallized one or more times after being first precipitated.

Regardless of the particular type of high solids process used in the process of the invention, the amine oxide product has acceptable color and, in fact, is substantially colorless. This permits the products to be used in applications, such as cosmetics, in which they could not be used if they had the intense color of the amine oxides formed by otherwise comparable processes in which the non-reactive gas is not used to dilute the carbon dioxide.

The following examples are given to illustrate the invention and are not intended as a limitation thereof.

EXAMPLES 1-4

Charge 100g of N-tetradecyldimethylamine and 0.5g of diethylenetriaminepentaacetic acid to a suitable reaction vessel. Stir the mixture and heat to 65°C while maintaining a gas sweep through the vapor space. Then add 23g of 70% aqueous hydrogen peroxide dropwise over a period of 10 minutes and raise the temperature to 75°C. Stir the reaction mixture at 75°C until the amine conversion reaches 99% as determined by proton NMR, adding a total of 40 mL of ethyl acetate as needed during the course of the reaction to maintain a stirrable reaction mixture. Then pour the reaction mixture into 300 mL of ethyl acetate and cool the resulting solution to 10°C to precipitate crystalline N-tetradecyldimethylamine oxide dihydrate. Recover the dihydrate by filtration. The results obtained by using different carbon dioxide/nitrogen mixtures as the gas sweep are shown in the table below, in which an intense orange color in represented by 0, pale yellow by Y, and white by W.

TABLE

| Example | 1 | 2 | 3 | 4 |
|---|---|---|---|---|
| Volume % $CO_2$ in gas sweep | 60 | 40 | 30 | 20 |
| Hrs. to 99% conversion | 2 | 3 | 3 | 4 |
| Product reaction mixture color | 0 | Y | Y | Y |
| Crystallized product color | 0 | W | W | W |

These results compare with (A) a reaction time of only two hours but an intense orange color in both the product reaction mixture and the crystallized product when the gas sweep is 100% carbon dioxide and (B) a reaction time of nine hours, a white product reaction mixture, and a white crystallized product when the reaction is conducted in an air atmosphere in the absence of carbon dioxide.

Claims

1. A process for preparing an amine oxide by reacting a tert-amine with hydrogen peroxide at a temperature of 20 - 100 °C in the presence of carbon dioxide and in a liquid medium that consitutes not more than 50 % of the weight of the reaction mixture, characterized in that discoloration of the product is minimized by conducting the reaction in the presence of an amount of non-reactive gas wherein the amount is such as to constitute 60 - 75 % of the combined volumes of non-reactive gas and carbon dioxide.

2. The process of claim 1 wherein the hydrogen peroxide is an aqueous solution having a concentration of at least 50 % by weight.

3. The process of claim 1 or 2 wherein the liquid medium comprises an organic solvent in which the tert-amine and amine oxide are soluble at the reaction temperatures but in which the amine oxide is insoluble at a lower temperature.

4. The process of claim 3 wherein the tert-amine is a compound corresponding to the formula RR′R″N in which R, R′ and R″ are primary alkyl groups containing 1 - 30 carbons.

5. The process of claim 4 wherein R is methyl or ethyl, R′ is an alkyl group containing 6 - 20 carbons, and R″ is independently selected from methyl, ethyl, and alkyl groups containing 6 - 20 carbons.

6. The process of claim 3 wherein (A) a stirrable reaction mixture of (1) a tert-amine corresponding to the formuly RR′R″N in which R is methyl or ethyl, R′ is an alkyl group containing 6 - 20 carbons, and R″ is

4

independently selected from methyl, ethyl and alkyl groups containing 6 - 20 carbons and (2) at least a stoichiometric amount of aqueous hydrogen peroxide is heated at a temperature of 20 - 100 °C in the presence of carbon dioxide and an amount of nitrogen such as to constitute 60 - 75 % of the combined volumes of carbon dioxide and nitrogen and in an organic solvent in which the tert-amine and amine oxide are soluble at the reaction temperatures but in which the amine oxide is insoluble at a lower temperature, (b) the water content of the reaction mixture is adjusted if desired, and (C) the reaction mixture is cooled to precipitate the amine oxide from the organic solvent.

7. The process of claim 6 wherein the water/amine oxide mol ratio in the reaction mixture that is cooled to precipitate the amine oxide is 1.9 - 2.1/1.

8. The process of claim 1 or 2 wherein the liquid medium is water.

9. The process of claim 8 wherein the tert-amine is a compound corresponding to the formula RR′R″N in which R is methyl or ethyl and R′ and R″ are independently selected from alkyl groups containing 6 - 20 carbons.


**Patentansprüche**

1. Verfahren zur Herstellung eines Aminoxids durch Reaktion eines tertiären Amins mit Wasserstoffperoxid bei einer Temperatur von 20 bis 100°C in Gegenwart von Kohlendioxid und in einem flüssigen Medium, das nicht mehr als 50 % des Gewichts der Reaktionsmischung ausmacht, dadurch gekennzeichnet, daß eine Verfärbung des Produkts dadurch minimiert wird, daß man die Reaktion in Gegenwart einer Menge eines nicht-reaktiven Gases durchführt, worin die Menge so groß ist, daß sie 60 bis 75 % der vereinigten Volumina von nicht-reaktivem Gas und Kohlendioxid ausmacht.

2. Verfahren nach Anspruch 1, worin das Wasserstoffperoxid eine wässrige Lösung ist, die eine Konzentration von wenigstens 50 Gew.-% hat.

3. Verfahren nach Anspruch 1 oder 2, worin das flüssige Reaktionsmedium ein organisches Lösungsmittel umfaßt, in dem das tertiäre Amin und das Aminoxid bei den Reaktionstemperaturen löslich sind, in dem jedoch das Aminoxid bei einer niedrigeren Temperatur unlöslich ist.

4. Verfahren nach Anspruch 3, worin das tertiäre Amin eine Verbindung ist, die der Formel RR′R″N entspricht, worin R, R′ und R″ primäre Alkylgruppen sind, die 1 bis 30 Kohlenstoffatome enthalten.

5. Verfahren nach Anspruch 4, worin R Methyl oder Ethyl ist, R′ eine Alkylgruppe ist, die 6 bis 20 Kohlenstoffatome enthält, und R″ unabhängig davon gewählt ist unter Methyl, Ethyl und Alkylgruppen, die 6 bis 20 Kohlenstoffatome enthalten.

6. Verfahren nach Anspruch 3, worin
   (A) eine rührbare Reaktionsmischung aus
      (1) einem tertiären Amin, das der Formel RR′R″N entspricht, worin R Methyl oder Ethyl ist, R′ eine Alkylgruppe ist, die 6 bis 20 Kohlenstoffatome enthält, und R″ unabhängig davon gewählt ist unter Methyl, Ethyl und Alkylgruppen, die 6 bis 20 Kohlenstoffatome enthalten, und
      (2) wenigstens einer stöchiometrischen Menge wässrigen Wasserstoffperoxids
   auf eine Temperatur von 20 bis 100°C in Gegenwart von Kohlendioxid und einer Menge an Stickstoff, die so groß ist, daß sie 60 bis 75% der vereinigten Volumina von Kohlendioxid und Stickstoff ausmacht, und in einem organischen Lösungsmittel aufgeheizt wird, in dem das tertiäre Amin und das Aminoxid bei den Reaktionstemperaturen löslich sind, in dem jedoch das Aminoxid bei einer niedrigeren Temperatur unlöslich ist,
   (B) der Wassergehalt der Reaktionsmischung eingestellt wird, sofern dies erwünscht ist, und
   (C) die Reaktionsmischung abgekühlt wird, um das Aminoxid aus dem organischen Lösungsmittel zu fällen.

7. Verfahren nach Anspruch 6, worin das Molverhältnis Wasser: Aminoxid in der Reaktionsmischung, die abgekühlt wird, um das Aminoxid auszufällen, 1,9 bis 2,1 : 1 beträgt.

**8.** Verfahren nach Anspruch 1 oder 2, worin das flüssige Medium Wasser ist.

**9.** Verfahren nach Anspruch 8, worin das tertiäre Amin eine Verbindung ist, die der Formel RR'R''N entspricht, worin R Methyl oder Ethyl ist und R' und R'' unabhängig voneinander gewählt sind unter Alkylgruppen, die 6 bis 20 Kohlenstoffatome enthalten.


**Revendications**

**1.** Procédé de production d'un oxyde d'amine par réaction d'une amine tertiaire avec du peroxyde d'hydrogène à une température de 20 à 100°C en présence d'anhydride carbonique et dans un milieu liquide qui ne constitue pas plus de 50 % du poids du mélange réactionnel, caractérisé en ce que le changement de couleur du produit est minimisé par la conduite de la réaction en présence d'une quantité de gaz non réactif, quantité qui est telle qu'elle constitue 60 à 75 % des volumes totaux de gaz non réactif et d'anhydride carbonique.

**2.** Procédé suivant la revendication 1, dans lequel le peroxyde d'hydrogène est une solution aqueuse ayant une concentration d'au moins 50 % en poids.

**3.** Procédé suivant la revendication 1 ou 2, dans lequel le milieu liquide comprend un solvant organique dans lequel l'amine tertiaire et l'oxyde d'amine sont solubles aux températures de réaction, mais dans lequel l'oxyde d'amine est insoluble à une température plus basse.

**4.** Procédé suivant la revendication 3, dans lequel l'amine tertiaire est un composé correspondant à la formule RR'R''N dans laquelle R, R' et R'' sont des groupes alkyle primaires contenant 1 à 30 atomes de carbone.

**5.** Procédé suivant la revendication 4, dans lequel R est le groupe méthyle ou éthyle, R' est un groupe alkyle contenant 6 à 20 atomes de carbone et R'' est choisi indépendamment entre le groupe méthyle, le groupe éthyle et des groupes alkyle contenant 6 à 20 atomes de carbone.

**6.** Procédé suivant la revendication 3, dans lequel (A) un mélange réactionnel pouvant être agité (1) d'une amine tertiaire correspondant à la formule RR'R''N dans laquelle R est un groupe méthyle ou éthyle, R' est un groupe alkyle contenant 6 à 20 atomes de carbone et R'' est choisi indépendamment entre le groupe méthyle, le groupe éthyle et des groupes alkyle contenant 6 à 20 atomes de carbone et (2) au moins une quantité stoechiométrique de peroxyde d'hydrogène aqueux, est chauffé à une température de 20 à 100°C en présence d'anhydride carbonique et d'une quantité d'azote constituant 60 à 75 % des volumes totaux d'anhydride carbonique et d'azote et dans un solvant organique dans lequel l'amine tertiaire et l'oxyde d'amine sont solubles aux températures de réaction mais dans lequel l'oxyde d'amine est insoluble à une température plus basse, (b) la teneur en eau du mélange réactionnel est ajustée le cas échéant et (c) le mélange réactionnel est refroidi pour précipiter l'oxyde d'amine dans le solvant organique.

**7.** Procédé suivant la revendication 6, dans lequel le rapport molaire de l'eau à l'oxyde d'amine dans le mélange réactionnel qui est refroidi pour précipiter l'oxyde d'amine est de 1,9 - 2,1/1.

**8.** Procédé suivant la revendication 1 ou 2, dans lequel le milieu liquide est l'eau.

**9.** Procédé suivant la revendication 8, dans lequel l'amine tertiaire est un composé correspondant à la formule RR'R''N dans laquelle R est un groupe méthyle ou éthyle et R' et R'' sont choisis indépendamment entre des groupes alkyle contenant 6 à 20 atomes de carbone.